(19) Europäisches Patentamt / European Patent Office / Office européen des brevets

(11) **EP 3 160 582 B1**

(12) **EUROPEAN PATENT SPECIFICATION**

(45) Date of publication and mention
of the grant of the patent:
**23.09.2020 Bulletin 2020/39**

(51) Int Cl.:
***A61N 1/40*** *(2006.01)*    ***A61N 1/32*** *(2006.01)*
***A61N 2/02*** *(2006.01)*

(21) Application number: **15734472.2**

(22) Date of filing: **25.06.2015**

(86) International application number:
**PCT/IB2015/054797**

(87) International publication number:
**WO 2015/198270 (30.12.2015 Gazette 2015/52)**

(54) **SYSTEM FOR THERAPEUTIC TREATMENTS WITH ELECTROMAGNETIC WAVES**

SYSTEM FÜR DIE THERAPEUTISCHEN BEHANDLUNG MIT ELEKTROMAGNETISCHEN WELLEN

SYSTÈME POUR TRAITEMENTS THÉRAPEUTIQUES AU MOYEN D'ONDES ÉLECTROMAGNÉTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2014 IT TO20140505**

(43) Date of publication of application:
**03.05.2017 Bulletin 2017/18**

(73) Proprietor: **Thereson S.r.l.
20871 Vimercate (MB) (IT)**

(72) Inventors:
• **FERMI, Enrico
I-20871 Vimercate (MB) (IT)**

• **ZANOTTI, Daniele
I-20871 Vimercate (MB) (IT)**

(74) Representative: **Meindl, Tassilo et al
Buzzi, Notaro & Antonielli d'Oulx S.p.A.
Corso Vittorio Emanuele II, 6
10123 Torino (IT)**

(56) References cited:
**FR-A1- 2 648 567        US-A- 5 830 140
US-A1- 2002 059 247    US-A1- 2004 193 065
US-A1- 2005 033 381    US-A1- 2005 116 742**

## Description

Technical field

The present disclosure relates to a system for therapeutic treatments with electromagnetic waves.

Description of the prior art

Document No. WO 2012/172504 describes a system that is able to subject the various types of biological tissue and/or the various organs involved in tissue damage and in consequent repair to a stimulus that will drive in magnetic resonance the cell structures of the aforesaid tissues and organs. In fact, it would appear that different tissues and organs respond, *in vivo,* to frequencies of weak electromagnetic fields that have the property of sending specific cell structures of those tissues or organs into resonance. These "characteristic" frequencies are, for example, those of the electroencephalogram, which have a frequency range that falls between 0.1 and 42 Hz. Likewise, also other organs, tissues, and cell structures have typical frequency ranges, which have the property of responding to "resonance stimuli", if exposed to magnetic fields pulsating at these frequencies.

According to the document No. WO 2012/172504, in order to determine a stimulus that is positive for tissue repair, it is necessary to obtain resonance effects from the different organs/tissues involved simultaneously.

Consequently, in general, there is posed the far from simple problem of generating in the anatomical part concerned a pulsating magnetic field that will possesses the frequency contents of the various characteristic ranges of each tissue (in general different from, but at times superimposable on, the typical band from 0.1 to 100 Hz) in a targeted way, since pulsating electromagnetic fields that have frequency ranges that are different or wider can have a low probability of producing the effect of therapeutic resonance. Furthermore, these electromagnetic fields will certainly supply frequencies that cause at the most a thermal or saturating stimulus, also by virtue of the fact that the amplitudes typically used in the known art are much wider than the useful ones, thus frequently causing an exchange of energy that may instead be harmful.

To be able to obtain an adequate waveform to create simultaneously series of well-defined frequencies only within certain ranges, the document No. WO 2012/172504 proposes an apparatus that generates an electromagnetic wave with specific contributions in frequency that is constituted by a cascade of pulses generated at a certain frequency. In particular, a given number of these base pulses or bursts (in a typical range of from 2 to 200 pulses) is generated within a packet, and the packets in turn are generated in a cascade of a certain number of these packets, which constitutes a "train" of packets. The frequency content of these pulses, packets, and trains is hence equivalent to obtaining the resonance frequencies, and those alone, that characterize the typical ranges of the various organs/tissues. Moreover, this structure of the signal not only enables the desired frequencies and only those to be obtained, but also enables their modification on one and the same apparatus in a simple and deterministic way, by adjusting the typical parameters of the components of the wave.

More specifically, the present invention relates to a system for therapeutic treatments with electromagnetic waves according to the preamble of Claim 1, which is known, e.g. from US2005/0116742 A1. Document US2005/0033381 A1 may also be of interest for the present invention.

Object and summary of the present disclosure

The inventors have noted that the apparatus described in the document No. WO 2012/172504 enables the characteristic frequencies to be obtained in a convenient and economically advantageous way because only a simple control circuit is required, which activates and deactivates the base pulse to obtain the packets and trains. However, experiments conducted by the present applicant have shown that the signal generated by this apparatus presents some drawbacks that adversely affect the therapeutic treatment, such as for example the following:

- the signal in any case presents low-amplitude contributions at undesired frequencies;
- the amplitude of the individual frequency peaks cannot be modified; and
- the frequency of the individual peaks cannot be modified during execution of a program.

The object of the present invention is to provide an apparatus for therapeutic treatments that overcomes one or more of the drawbacks mentioned above.

According to the present invention, the above object is achieved by a system having the characteristics that form the subject of Claim 1.

The claims form an integral part of the technical teaching provided herein in relation to the invention.

In various embodiments, the system for therapeutic treatments with electromagnetic waves comprises a wave generator that is configured for generating a driving signal for a diffuser, which emits the electromagnetic wave used for the treatment.

In various embodiments, the system comprises a digital-to-analog converter configured for receiving at input digital data and for supplying at output the driving signal. The system further comprises a memory that contains discretized data that identify the spectrum of the driving signal in the frequency domain. For instance, in various embodiments, the discretized data that identify the spectrum of the driving signal in the frequency domain comprise data that identify a list of a plurality of harmonics that the driving signal should comprise. Furthermore, in various embodiments, these data also identify the respective amplitudes of the harmonics.

In various embodiments, at least one digital processing unit receives the discretized data that identify the spectrum of the driving signal and converts the discretized data in the frequency domain into discretized data that identify the waveform of the driving signal in the time domain. For instance, in various embodiments, the processing unit determines a sequence of digital samples via calculation of a sum of sinusoidal waves, where the sinusoidal waves have the frequencies of each the harmonics.

In various embodiments, the digital processing unit hence sends the discretized data that identify the waveform of the driving signal to the digital-to-analog converter.

In various embodiments, the conversion can be calculated in real time. In this way, the digital processing unit can vary the spectrum of the signal during execution of a treatment program.

For instance, in various embodiments, the system comprises a sensor that supplies a biofeedback signal, and the digital processing unit varies the spectrum as a function of the biofeedback signal.

Brief description of the drawings

Embodiments of the present disclosure will now be described in detail with reference to the attached drawings, which are provided purely by way of nonlimiting example and in which:

- Figures 1, 2, 3, 7, and 8 show possible embodiments of a treatment system according to the present description;
- Figures 4, 5, and 9 are flowcharts that show possible embodiments for generation of a driving signal for the systems illustrated in Figures 1, 2, 3, and 6; and
- Figures 6 and 10 show some details of generation of a driving signal according to the present disclosure.

Detailed description of embodiments

In the ensuing description various specific details are illustrated aimed at providing an in-depth understanding of the embodiments. The embodiments may be obtained without one or more of the specific details, or with other methods, components, materials, etc. In other cases, known structures, materials, or operations are not illustrated or described in detail so that various aspects of the embodiments will not be obscured.

Reference to "an embodiment" or "one embodiment" in the framework of the present description is intended to indicate that a particular configuration, structure, or characteristic described in relation to the embodiment is comprised in at least one embodiment. Hence, phrases such as "in an embodiment" or "in one embodiment" that may be present in various points of this description do not necessarily refer to one and the same embodiment. Furthermore, particular conformations, structures, or characteristics may be combined in an adequate way in one or more embodiments.

The references used herein are provided merely for convenience and hence do not define the sphere of protection or the scope of the embodiments.

As mentioned previously, the system according to the present disclosure is configured for generating an electromagnetic wave with specific frequency contributions.

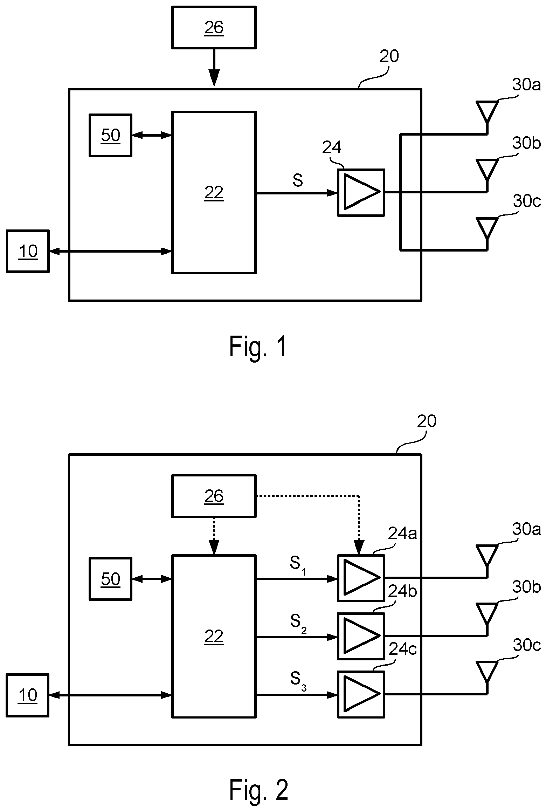

Figure 1 shows a possible embodiment of a system for therapeutic treatments according to the present description.

In the embodiment considered, the system comprises an apparatus 20 that generates at least one driving signal for at least one diffuser 30, which emits a corresponding treatment signal.

In particular, in the embodiment considered, the apparatus 20 comprises a control circuit 22 configured for generating a signal S that is sent through a power amplifier 24 to at least one antenna or diffuser 30. For instance, in the embodiment considered, three antennas 30a, 30b, and 30c are illustrated. For instance, in various embodiments, the number of the diffusers 30 is between two and twenty.

As mentioned previously, typically the electromagnetic field issued by a diffuser 30 will be between 40 nT and 100 $\mu$T.

In general, the diffusers 30 are built for specific applications or treatments and may be in different forms, such as for example in the form of pads or mats, which may be plane or modelled anatomically to reproduce the area of application, or handpieces.

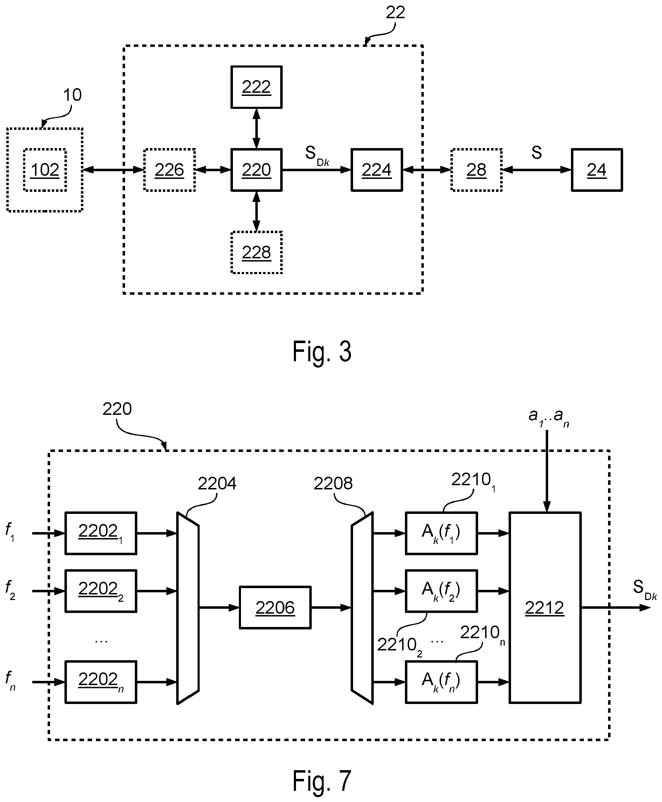

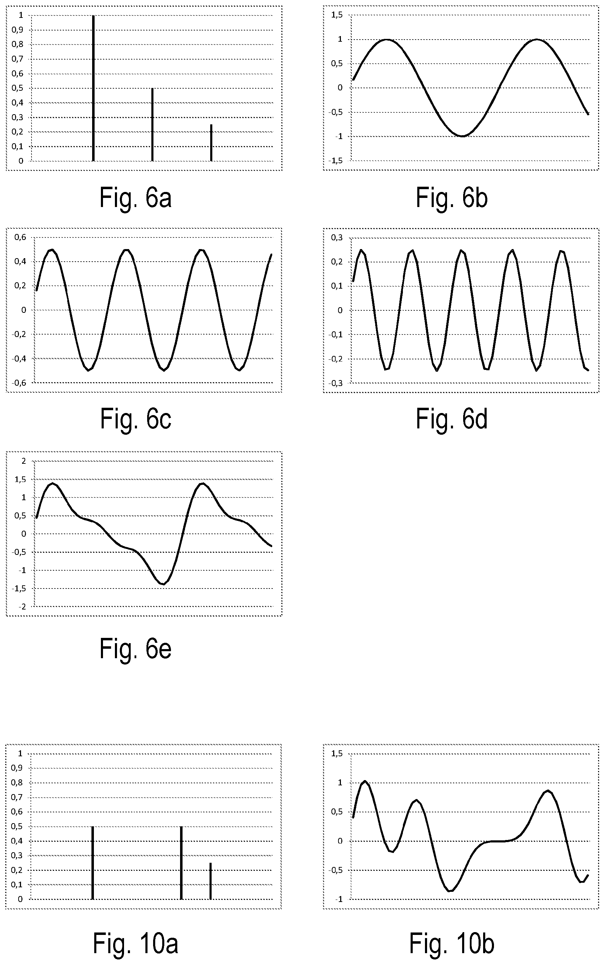

Typically, the above diffusers are obtained via plane solenoids that are applied on a support. For instance, the diffusers illustrated in Figures 6 and 7 of the document No. WO 2012/172504, may be used for this purpose.

Consequently, in the embodiment illustrated in Figure 1, the control circuit 22 generates only a single driving signal S, which is sent by means of an amplifier 24 to one or more diffusers 30; i.e., the diffusers are driven with the same signal S. In general, also a plurality of amplifiers may be provided, for example one amplifier could be provided for each diffuser 30, and each amplifier 24 could be set with a different amplification coefficient.

Instead, Figure 2 shows a currently preferred embodiment, in which the control circuit 22 generates a plurality of driving signals S. For instance, in the embodiment considered, the control circuit 22 generates three driving signals $S_1$, $S_2$, and $S_3$ that are sent through respective amplifiers 24a, 24b, and 24 to three diffusers 30a, 30b, 30c; i.e., each diffuser 30 is driven by a respective driving signal S and via a respective amplifier 24.

In general, the embodiments illustrated in Figures 1 and 2 may also be combined in such a way that a plurality of diffusers 30 is driven via at least one driving signal S, where for each driving signal S at least one amplifier 24 is provided and where each amplifier 24 can have a fixed or configurable amplification coefficient.

For instance, in one embodiment, at least two driving signals $S_1$ and $S_2$ are used. In particular, the first driving signal $S_1$ is used for a stimulation with systemic effect. For this purpose, a diffuser is typically used, designed to emit electromagnetic radiation on a particular system of the person treated, such as for example the endocrine system, the immune system, the lymphatic system, or the muscular system. Instead, the second driving signal $S_2$ is used for a stimulation with local effect. For this reason, the first signal $S_1$ also comprises the characteristic frequencies of the system to be treated, whereas the second signal $S_2$ comprises the characteristic frequencies of the local area to be treated, for example of the tissue or organ.

In Figures 1 and 2 a block 26 is moreover shown, which represents a supply circuit, such as for example an electronic (DC/DC or AC/DC) converter and/or a battery. In general, this supply circuit 26 may be incorporated in the apparatus 20 (see Figure 2) or be provided at least in part externally (see Figure 1).

In various embodiments, the system further comprises an external processing unit 10, such as for example a PC, which is designed to communicate with the apparatus 20, for instance for configuring the apparatus 20 and/or for downloading a treatment protocol from the apparatus 20.

Finally, in various embodiments, the apparatus 20 may comprise a user interface 50, which includes, for example, a plurality of keys and/or state indicators, a touch screen, etc.

Figure 3 shows a possible embodiment of the control circuit 22.

In the embodiment considered, the control circuit 22 comprises at least one digital processing unit 220, a memory 222, and a digital-to-analog converter (DAC) 224. For instance, the processing unit 220 may be a microprocessor, a digital signal processor (DSP), a field-programmable gate array (FPGA), a dedicated integrated circuit, or a combination of these. Instead, the memory 222 is preferably a nonvolatile memory, such as for example an EEPROM (electrically erasable programmable read-only memory) or a flash memory.

In various embodiments, the control circuit 22 further comprises a communication interface 226, which enables exchange of information with the external processing unit 10. For instance, the communication interface 224 may be an RS-232, USB (universal serial bus) interface or even LAN (local-area network) or WAN (wide-area network) cards for wired or wireless communication.

In general, the characteristic data of at least one driving signal S are stored in the memory 222. The processing unit 220 reads these data from the memory 222 and generates one or more digital driving signals $S_D$ that are converted into respective analog driving signals S through the DAC or DACs 224.

For instance, in one embodiment a DAC is used that has at least a 10-bit resolution, preferably a 16-bit or 24-bit resolution, and a sampling rate of at least 32 kHz. For instance, in a preferred embodiment a 24-bit 384-kHz DAC is used.

In various embodiments, the apparatus further comprises at least one active or passive lowpass filter 28 that filters the driving signal S. For instance, this filter may filter the signal at output from the converter 224 and/or the amplifier 24.

As mentioned previously, the maximum frequency for the therapeutic treatments is typically lower than 2000 Hz. For this reason, also the above filter 28 typically has a cutoff frequency of between 50 Hz and 2 kHz, preferably between 250 and 500 Hz.

In general, the cutoff frequency of the filter 28 may also be configurable. For instance, in one embodiment, the control circuit 22, for example the digital processing unit 220, may determine the maximum frequency for a given driving signal S, for example as a function of the data that are stored in the memory 222, and configure the filter 28 appropriately.

In general, this filter 28 is purely optional, because filtering could be obtained also via an appropriate sizing of the diffuser 30.

Consequently, the therapeutic-treatment system according to the present disclosure comprises a processing circuit that receives data that identify the spectrum of at least one driving signal S used to drive a diffusor 30, which emits a corresponding electromagnetic wave used for the treatment. In general, this processing circuit comprises at least one digital processing unit 202 inside the apparatus 20 and possibly an external processing unit 10, which may be connected even only temporarily to the apparatus 20, for example during an initial configuration step.

In general, the therapeutic-treatment system according to the present disclosure enables arbitrary specification of the spectrum of each driving signal S. In various embodiments, the system is configured for transforming this spectrum in the frequency domain into a discrete signal $S_D$ in the time domain, i.e., a series of digital samples. In fact, in various embodiments, this transformation is carried out via digital processing (and not through generators of analog signals). Furthermore, in various embodiments, the above transformation is calculated in real time in such a way that all the frequency contributions can vary selectively during execution of a treatment program.

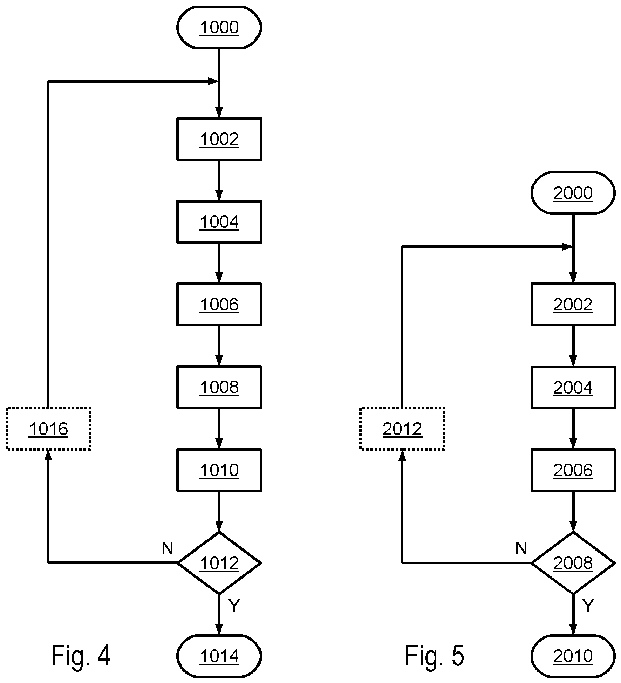

Figure 4 shows a first embodiment for generation of a driving signal S. The person skilled in the art will appreciate that the same scheme can also be extended to a larger number of driving signals S.

After a start step 1000, the processing circuit receives, in a step 1002, the data that identify the spectrum of the driving signal S.

For instance, in one embodiment, these data comprise the amplitudes of a given number of samples in the frequency domain between a minimum frequency and a maximum frequency, for example between 0 and 2 kHz, preferably between 0 and 500 Hz.

In a step 1004, the processing circuit transforms the above data from the frequency domain into the time domain. For instance, in one embodiment, the processing circuit is configured for carrying out an inverse discrete Fourier transform (IDFT). Consequently, the result of the transformation is a given number of digital samples $S_{Dk}$ in the time domain that specify the waveform of the signal S in time. In fact, since the data identify in discretized form the spectrum of the signal between a minimum frequency and a maximum frequency, the result of the transformation corresponds to a periodic signal that comprises a given number of digital samples $S_{Dk}$.

In a step 1006, the processing circuit stores the above digital samples $S_{Dk}$ in a memory. In general, these digital samples $S_{Dk}$ may be stored also in compressed form. For instance, in one embodiment only the differential values between two consecutive samples are stored.

In a first embodiment, steps from 1002 to 1006 are executed by the external processing unit 10. In this case, the external processing unit 10 comprises a memory 102 in which the data that identify the spectrum of the signal S are stored. Once these data have been transformed from the frequency domain into the time domain, the unit 10 saves, in step 1006, these data through the interface 226 in the nonvolatile memory 222 of the circuit 22. Consequently, in this embodiment, the memory 222 contains discretized data, i.e., the samples $S_{Dk}$, which identify the waveform of the signal S in time.

Instead, in a second embodiment, the processing unit 10 is only used for saving, by means of the interface 226, the data that identify the spectrum of the driving signal S in the nonvolatile memory 222, and steps from 1002 to 1006 are already executed by the processing unit 220. Consequently, in this embodiment, the memory 222 contains discretized data that identify the spectrum of the signal S in the frequency domain.

For instance, in various embodiments, the processing unit 220 stores, in step 1006, the result of the transform, i.e., the digital samples $S_{Dk}$ that represent the time plot of the digital driving signal S, in a second memory 228, such as for example a RAM. However, in general, the processing unit 220 could store the aforesaid digital samples $S_{Dk}$ also in the nonvolatile memory 222.

Consequently, irrespective of whether the transform has been calculated via the external unit 10 or the internal unit 220, at the end of step 1006, a memory of the apparatus 20 (the memory 222 or the memory 228) comprises discretized data that identify the time plot of the signal S, i.e., the samples $S_{Dk}$ in the time domain.

Next, in a step 1008, the processing unit 220 reads the digital samples $S_{Dk}$ from the memory 222 or 228, and sends, in a step 1010, these data to the DAC 224.

In the embodiment considered, the processing unit moreover verifies, in a step 1012, whether a treatment time (or a given number of iterations) has been reached. Also this datum is preferably configurable.

In the case where the treatment time has not elapsed (output "N" from the verification step 1012), the method continues to repeat steps 1008 and 1010.

Instead, in the case where the treatment time has elapsed (output "Y" from the verification step 1012), the method terminates, in a step 1014.

Consequently, in the embodiment considered, the transform (step 1004) of the spectral data is precalculated and saved in a memory (step 1006). Next, the driving signal S is generated in real time, by simply reading the digital samples from the memory (steps 1008 and 1010).

In general, in particular in the case of an implementation via dedicated hardware, the processing unit 220 may comprise for this purpose a number of processors that can also work in parallel. For instance, the memory that contains the digital samples $S_{Dk}$ may be a two-port memory. In this case, a first processor can calculate the transform (step 1004) and writes the data in the memory (step 1006) through the first port, whereas a second processor can read the data from the memory (step 1008) through the second port and generate the driving signal S (step 1010).

Instead, Figure 5 shows an embodiment that enables real-time transformation directly within the control circuit 22.

In this context, the inventor has noted that for therapeutic treatment with electromagnetic waves it is preferable for the driving signal S to comprise only a limited number of frequencies, and only those. For instance, the number of these characteristic frequencies is typically between two and fifty, preferably between three and twenty.

For this reason, in a currently preferred embodiment, the memory 222 contains a number n of frequencies $f_1 ... f_n$ that the driving signal S at output from the converter 224, and accordingly also the signal emitted by the diffusor 30, should comprise, as well as the respective amplitudes $a_1 ... a_n$. For instance, in one embodiment, a list of frequencies $f_1 ... f_n$ is directly saved in the memory 220, for example with a resolution of at least 16 bits, preferably 32 bits, which enables specification of frequencies in a given range, for example between 0 and 2 kHz, and the respective amplitudes $a_1 ... a_n$, for example with a resolution of at least 8 bits, preferably 16 bits.

In this embodiment, after a start step 2000, the processing unit 220 reads, in a step 2002, the characteristic data from the memory 222, i.e., the list of frequencies $f_1 ... f_n$ and the respective amplitudes $a_1 ... a_n$.

In the embodiment considered, the processing unit 220 then calculates in real time, in a step 2004, the digital sample $S_{Dk}$ for the current sampling instant $k$.

In particular, in one embodiment, the processing unit 220 calculates the composition of sinusoidal signals for the frequencies $f_1 \dots f_n$ required.

For instance, Figure 6a shows an example where the driving signal S must contain only the frequencies $f_1 = 10$ Hz, $f_2 = 20$ Hz, and $f_3 = 30$ Hz with the respective amplitudes $a_1 = 1$, $a_2 = 0.5$, and $a_3 = 0.25$. In this context, Figures 6b, 6c, and 6d show, respectively, sinusoidal waves that have the frequencies $f_1 = 10$ Hz, $f_2 = 20$ Hz, and $f_3 = 30$ Hz, and the amplitudes $a_1$, $a_2$, and $a_3$, respectively.

In this case, the signal S can be determined in time $t$ (which depends upon the instant $k$ and the sampling rate) as the sum of these three sinusoidal signals, i.e., in general:

$$S = \sum_{i=1}^{n} a_i \sin 2\pi f_i t$$

For instance, Figure 6e shows the driving signal S that results from the combination of the sinusoidal signals of Figures 6b to 6d.

In general, for each of these frequencies $f_1 \dots f_n$, i.e., sinusoidal signals, a respective phase offset could also be specified.

Consequently, thanks to the use of sinusoidal signals, the driving signal S will only have contributions at the frequencies $f_1 \dots f_n$.

Figure 7 shows an embodiment of the processing circuit 220 that is particularly useful for a dedicated hardware implementation (for example, FPGA or integrated circuit). However, in general, a similar architecture could also be implemented via corresponding software instructions.

In particular, in the embodiment considered the DDS (direct digital synthesis) approach is used for calculation of a single sinusoidal wave. In particular, DDS indicates as a method for generating, using digital electronics, an arbitrary periodic waveform starting from a single reference oscillator. Basically, in this case a LUT (look-up table) is used, obtained for example via a nonvolatile memory, in which the amplitudes $A_k$ corresponding to a given number of samples of a standard sinusoidal wave with a frequency $f_s$ are stored:

$$A_k = \sin 2\pi f_s t$$

For instance, in one embodiment, the frequency $f_s$ corresponds to the minimum frequency envisaged for the therapeutic treatments, for example 0.1 Hz.

Instead, for effective generation of the sinusoidal signal at the desired frequency, a counter is used that represents a phase accumulator, which is incremented by a certain value at each iteration, where the value of the increment substantially depends upon the ratio between the frequency $f_i$ of the required sinusoidal signal and the frequency $f_s$ of the standard sinusoidal signal.

For instance, as illustrated in Figure 7, $n$ counters $2202_1 \dots 2202_n$ may be provided, which receive as input the respective frequencies $f_1 \dots f_n$ and supply at output respective count values.

Consequently, to determine the amplitude $A_k(f_i)$ of a sinusoidal wave at the desired frequency $f_i$ at a given instant $k$, it is sufficient to use the value of the respective counter 2202, i.e., the phase, as address for the LUT.

As illustrated in Figure 7, it is not necessary for there to be provided a LUT 2206 for each counter 2202, but there could even be provided just a single LUT 2206, which receives sequentially, for example through a multiplexer 2204, one of the count values of the counters 2202. In this case, the result, i.e., the respective amplitude $A_k(f_i)$, can be stored, for example by means of a demultiplexer 2208, in respective registers $2210_1 \dots 2210_n$.

Finally, to determine the sample $S_{Dk}$, it is possible to calculate, for example by means of a circuit 2230, only the sum of the amplitudes $A_k(f_i)$, possibly weighted with the desired amplitudes $a_i$, as follows:

$$S_{Dk} = \sum_{i=1}^{n} a_i A_k(f_i)$$

Consequently, in the embodiment illustrated in Figure 5, the digital samples $S_{Dk}$ of the driving signal S are calculated in real time, i.e., for each instant $k$, and the respective digital sample is sent, in a step 2006, to the converter 224.

Also in this case, the processing unit 220 can verify, in a step 2008, whether a given treatment time has elapsed.

In the case where the treatment time has not elapsed (output "N" from the verification step 2008), the procedure continues to repeat steps 2004 and 2006.

Instead, in the case where the treatment time has elapsed (output "Y" from the verification step 2008) the method terminates, in a step 2010.

In general, for some applications it may even be superfluous to specify different amplitudes $a_1 \dots a_n$ for the various frequencies $f_1 \dots f_n$, and consequently this datum is purely optional and all the frequencies $f_1 \dots f_n$ can have the same amplitude.

Consequently, in general, the memory 222 within the control circuit may contain data that indicate the time evolution or the spectral characteristics of the driving signal S.

Furthermore, in various embodiments, these data in the memory 222 may be modifiable. For instance, as described previously, these data can be received through the interface 226. For instance, in this case, a user can select the spectral characteristics of the driving signal S through a software that is run on an external PC 10. Then this software can:

- in the case where the processing unit is able to transform the spectral data into the waveform of the driving signal S in the time domain, store these data directly in the memory 222 through the interface 226; or

- transform the data, for example via IDFT, into the time domain, and save the digital samples $S_{Dk}$ that identify the waveform of the signal S in the memory 226.

In various embodiments, the spectral data can also be configured directly through the user interface 50 of the apparatus 20. For instance, the user interface 50 could enable selection of different treatment programs with respective spectral characteristics. Furthermore, the user interface could also enable direct configuration of a plurality of characteristic frequencies $f_1 ... f_n$ of the driving signal S and/or the respective amplitudes $a_1 ... a_n$, i.e., the peaks in the frequency domain.

In general, the apparatus 20 could also be configured for enabling setting (via the communication interface 226 and/or the user interface 50) of additional configuration data, such as for example:

- a time or a number of iterations after which the control unit 22 inverts the sign of the driving signal S;
- an offset, i.e., a DC component in the driving signal S;
- data that configure the control unit in such a way that the driving signal S is rectified, by inversion of the negative half-waves, or else translated in amplitude to render it all positive or all negative; and/or
- a pause period, for example after an entire iteration of all the digital samples $S_{Dk}$.

The advantage of the system described hitherto lies precisely in the fact that the driving signal comprises only some characteristic frequencies that can be specified arbitrarily in the frequency domain. Furthermore, the main part of processing of the driving signal S is carried out in a digital way. In particular, in various embodiments, the digital samples $S_{Dk}$ are only converted via a converter 224 into an analog signal S, and this driving signal S is sent to at least one diffuser 30 only via at least one power amplifier 24 and possibly one or more filters 28; i.e., the driving signal S is no longer combined with other analog signals. Consequently, there is no need for a number of analog-signal generators and/or analog modulators.

Furthermore, the characteristic frequencies of the driving signal can vary in real time. For instance, the embodiment described with reference to Figure 5 intrinsically works in real time. Consequently, a step 2012 may be envisaged, in which the frequencies $f_1 ... f_n$ and/or the amplitudes $a_1 ... a_n$ are adapted for calculation of the next sample $S_{Dk}$. Also in the embodiment described with reference to Figure 4 a step 1016 may be envisaged that updates the samples $S_{Dk}$ between two iterations of the digital samples $S_{Dk}$.

In fact, the inventors have noted that for some treatments the system may be required to vary, during execution of the program, the amplitude and/or frequency of some characteristic peaks in the spectrum.

In fact, in the literature and on the basis of experiments conducted by the present applicant there exist at least four possible hypotheses of interaction between low-frequency and low-intensity pulsed electromagnetic fields and the biological tissue:

a) action on RNA polymerase and stimulation of cell replication;

b) action on ATP synthesis and hydrolysis;

c) effectiveness of the reduction-oxidation processes; and

d) action on opening of voltage-dependent calcium channels and improvement of cell metabolism, in particular with an effect of increased affinity and effectiveness of the membrane receptors coupled to G proteins.

The above hypotheses are summarized hereinafter.

RNA polymerase

It has been suggested by different authors that nonlinear excitations, in particular solitary waves, could play a fundamental role in the functional process of transcription of DNA (deoxyribonucleic acid), bringing about opening of the double chain necessary for the RNA (ribonucleic acid) polymerase to be able to copy the genetic code.

The first step in gene expression is transcription of the DNA from the original model, contained in the cell, to a copy - messenger RNA - that will then be used as "master copy" in determining the protein sequences in conformance with the genetic information. The evolutionary advantage of such a messenger is evident: in this way, the original DNA is "open" - and hence less protected - for the shortest time possible.

The transcription process appears to be carried out by a specialized enzyme, RNA polymerase (RNAP). The dynamic aspect of transcription can be summarized as follows: the RNAP opens a region of the DNA, which has the size of approximately 15 to 20 base pairs, the socalled "transcription bubble", and then travels along the DNA chain maintaining the size of the open region more or less constant, simultaneously opening the chain in front of it and closing the chain behind it. The RNAP travels along the DNA chain at a rate of several tens or hundreds of base pairs per second (different authors give different estimates). Since each base pair is connected by two or three hydrogen bonds, the energy involved in this process, even considering just the energy required for opening (and closing) the DNA chain, should be of the order of at least hundreds, if not thousands, of H-bonds per second.

Biophysics suggests the existence of nonlinear excitations that travel along the DNA chains, causing local opening of the double chain. The RNAP could then shift thanks to these vibrational waves and exploit the fact that the chain is already opened to read the DNA sequence without consuming the energy that would instead be used for opening the double chain.

Another advantage of this theory regards closing of the double chain by the RNAP after its passage: if this were to happen in a non-coordinated way, it would generate a considerable amount of random movement and hence of thermal energy; since, instead, it corresponds to overcoming a nonlinear excitation (which could be called "nonlinear coherent structure"), and no thermal energy will be generated.

The transcription bubble could hence correspond to a solitary wave ("soliton") travelling along the chain that the RNAP could use to be able to access the sequence of bases without expending any energy for opening the double helix.

The hypothesis underlying the interaction between the weak magnetic field with specific contributions in frequency, with the biological tissues, could hence be linked to the action produced by the field itself on the cells, in the form of elastic vibration: a rotational twisting moment acts on the topology of the DNA, with the effect of stimulation of gene expression. In this case, the temporal variation of the magnetic field, and not its intensity, would be the basis of the effectiveness of the signal according to the present description.

## Action on synthesis and hydrolysis of ATP

According to this hypothesis, the electrical effect that accompanies the magnetic field affects the distribution of charges, i.e., biasing, of the mitochondrial membranes with an effect on the capacity of production of ATP (adenosine triphosphate) and its use.

In general, also the aspect described hereinafter, regarding the flow of current of Ca++ ions, affects synthesis and use of ATP.

## Effectiveness of the reduction-oxidation processes

At the systemic level, there may be noted a reduction of oxidative stress and increase of antioxidant defences, with the final effect of improvement of the inflammatory state.

At the cell level, it has been noted how some frequencies reduce free radicals, for example, in endothelial cells (see, for example, Sai Ma *et al.,* "Protective Effects of Low-Frequency Magnetic Fields on Cardiomyocytes from Ischemia Reperfusion Injury via ROS and NO/ONOO", Oxidative Medicine and Cellular Longevity, 2013).

## Action on opening of voltage-dependent calcium channels and improvement of cell metabolism

Improvement of cell metabolism is typically expressed at different levels, for example:

- the increase of intracellular calcium entails restoration of cell-communication functions and synthesis and use of ATP, as expression of the work of mitochondria; or
- use of glucose by the cell is improved thanks to the increased affinity of the membrane receptors coupled to G proteins, amongst which also insulin receptors.

Insulin appears to regulate the metabolism and gene expression. The main function is to promote glucose uptake by the cells of numerous tissues, activating glycolysis, which is responsible for the production of energy, and cell proliferation, by stimulation of DNA replication, in addition to regulating the concentration of glucose in the blood. When the cells reduce their own sensitivity to the action of insulin, the insulin-resistance mechanism is triggered: the hormone, in this case, would appear to produce a biological effect that is inferior to expectations.

According to this theory, the effect of interaction of the electromagnetic field according to the present disclosure on insulin receptors, by increasing the effectiveness and affinity thereof, reduces insulin-resistance, improving glucose uptake and metabolic processes. In fact, in subjects affected by hyperglycaemia there may be noted a reduction of the glycaemic values and a better metabolic control.

For instance, an action of the low-intensity and low-frequency magnetic field that favours the increase of the flow of current of calcium ions in cells in general is described (e.g., Pall M.L., "Electromagnetic fields act via activation of voltage-gated calcium channels to produce beneficial or adverse effects", J. Cell. Mol. Med. 2013 Aug.; 17(8):958-65. doi: 10.1111/jcmm.12088. Epub 2013, Jun. 2). In the specific case of the pancreatic beta cells, the increase of Ca++ intracell current could stimulate insulin release (Aruna S. Rajan et al. "Ion Channels and Insulin Secretion", Diabetes Care, 13: 340-63, 1990).

Consequently, irrespective of the possible explanations of efficacy of the therapeutic treatment with low-intensity electromagnetic waves according to the present disclosure, the signal directly affects the tissue treated, and for this reason the characteristics of the tissue treated and/or the change of the dynamic state of the tissue treated following upon exposure to the electromagnetic wave should be taken into consideration. For instance, it seems that the dynamic variation of the harmonics prevents the phenomenon of cell adaptation, with reduction of the sensitivity to the stimulation induced and of the consequent responses. Adaptation can be defined in fact as the reduction of the response in conditions of constant stimulation.

In general, these variations of the treatment signal S may be made according to a predetermined program and/or as a function of a feedback signal. Furthermore, the variation may be made via the external processing unit 10 or the control circuit 22, for example the processing unit 220.

As explained previously, the system according to the present disclosure will generate an electrical signal S for driving the diffusers 30 on the basis of spectral content, which can be specified arbitrarily directly in the frequency domain.

Typically only a limited number of frequency peaks, i.e., harmonics, is selected, where these harmonics can be selected, for example, on the basis of:

- the user's choice (typically for research purposes);
- predefined models available in the scientific literature, for example characteristic frequencies of the tissue treated;
- theoretical calculations obtained via application of mathematical formulas useful for description of the interaction between the biological tissue and the electromagnetic field, such as calculation of the ion cyclotron resonance that identifies the frequency $f_c$ of an alternating magnetic field that is applied to an ion with a given charge-to-mass ratio $q/m$ subjected to a static magnetic field $B$ (see, for example, Halliday, D. and Resnick, R., "Fundamentals of Physics", 2nd ed., Wiley, New York, 1981) :

$$f_c = \frac{q}{2\pi m} \quad B$$

Considering in this case, for example, the $q/m$ ratio for potassium, which is 2.45 x $10^6$ C/kg, and imposing a value of $B$ equal of 41 $\mu$T, a value of frequency $f_c$ = 16 Hz would be obtained.

Consequently, the system according to the present disclosure enables arbitrary variation of the harmonics selected during application of the therapy, both as frequency values and as amplitude (intensity).

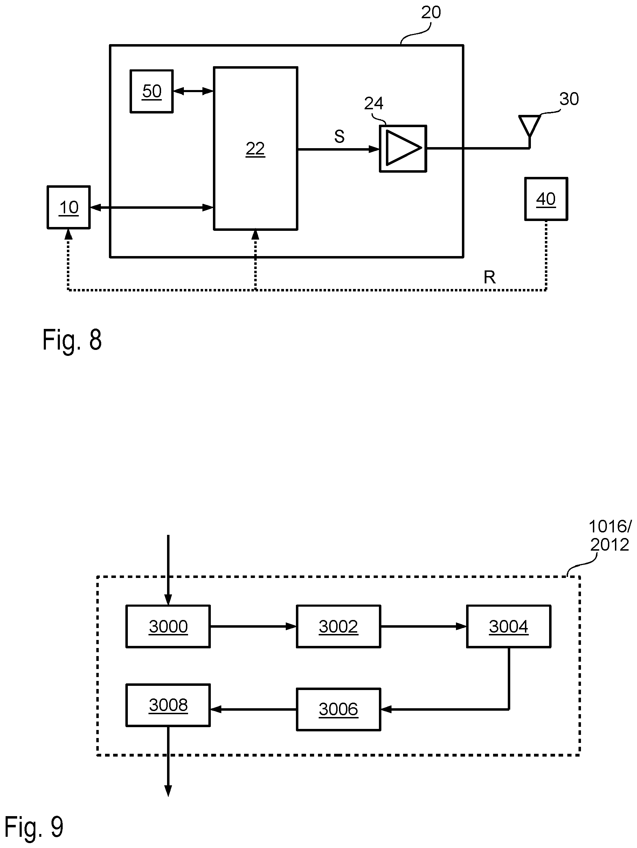

For instance, Figure 8 shows an embodiment in which the treatment system comprises at least one sensor 40.

In particular, in the embodiment considered, the system is able to adapt the contributions in frequency (amplitude and/or frequency) of the driving signal S in real time (see steps 1016 and 2012, described previously) as a function of at least one biofeedback signal R, i.e., data that identify certain biological parameters of the tissue or organ being treated.

For instance, in various embodiments, the above sensor 40 may be a temperature sensor, preferably a passive IR temperature sensor, configured for detecting the temperature of the area being treated. In general, other sensors may also be used configured for detecting parameters of the tissue being treated, such as for example muscular tension, skin temperature, skin conductance, arterial blood pressure, and/or heart rate. For instance, for this purpose, detection by electromyography may be used, and/or sensors that detect peripheral vasoconstriction, electrodermal activity, etc.

In a preferred embodiment, at least one sensor 40 is used, configured for detecting the amount of nitric oxide. For instance, for this purpose it is possible to use a nitric-oxide sensor such as the one described in the document No. US 7,897,399 or else commercial products, for example ones manufactured by Harvard Apparatus, 84 October Hill Road, Holliston, Massachusetts 01746, USA or by Innovative Instruments, Inc., 8533 Queen Brooks Ct., Tampa, Florida 33637, USA.

In one embodiment, the signal or signals R coming from the sensor or sensors 40 is used for varying the spectral content of the signal S, prevalently as a function of its or their variations in amplitude.

For instance, in one embodiment, the external processing unit 10 and/or the control circuit 22 may be configured for varying the value of amplitude and/or frequency of one or more harmonics constituting the signal S to verify whether this modification increases or decreases the value of a feedback signal R. In this way, it is possible to set a combination of frequencies $f_1 \dots f_n$ and/or amplitudes $a_1 \dots a_n$ of the signal S that minimizes or maximizes a feedback signal R.

For instance, in one embodiment, the external processing unit 10 and/or the control circuit 22 are configured for increasing the value of frequency of at least one of the frequencies $f_1 \dots f_n$ of the signal S. In the case where this reduces skin conductivity, the system uses the new frequencies. Instead, in the case where the value of amplitude of the skin conductivity were to increase, the system can return to the harmonics that constituted the starting signal S and test the effect of reduction of the frequencies. The same approach could be used also for choosing the respective amplitudes $a_1 \dots a_n$.

In one embodiment, also in a similar way for the signal R measured by the nitric-oxide sensor, the system can increase and/or decrease the value of at least one of the frequencies $f_1 \dots f_n$ and/or amplitudes $a_1 \dots a_n$ of the signal S and monitor the variation of the amount of nitric oxide.

In general, it is also possible to use a sensor 40 configured for detecting the electromagnetic field emitted by the diffusers 30 that results after its interaction with the biological tissue, i.e., the response of the biological tissue to stimulation. In this way, it is possible to determine the characteristic frequencies of the biological tissue being treated, and possibly shift some frequencies to improve the resonant effect.

Consequently, the sensor 40 illustrated in Figure 8 may be a measuring antenna 40, such as for example a solenoid. In particular, in various embodiments, the aforesaid measuring antenna 40 is configured for detecting the electromagnetic field that is re-emitted by the biological tissue. For instance, for this purpose the driving signal S can be interrupted periodically, and the electromagnetic field emitted by the biological tissue can be measured.

Figure 9 shows, in this context, a possible embodiment of steps 1016 and 2012 illustrated in Figures 3 and 4.

In the embodiment considered, the control circuit 22 or the external processing unit 10 detects, in a step 3000, the time plot of the signal R detected by the measuring antenna 40 for a given time interval, for example for a cycle of iteration of the digital samples $S_{Dk}$, and stores, in a step 3002, respective digital samples $R_{Dk}$ in a memory, such as for example the memory 228.

Next, the control circuit 22 or the external processing unit 10 possibly filters the digital samples $R_{Dk}$, for instance with a lowpass digital filter, and determines, in a step 3004, the spectrum in the frequency domain for the aforesaid signal R, for example applying a discrete Fourier transform (DFT), for example the fast Fourier transform (FFT), in such a way as to detect the frequency peaks in the spectrum of the signal R. In general, there may also be provided an amplifier that amplifies the signal coming from the antenna 40 and/or an analog filter.

In a step 3006, the control circuit 22 or the external processing unit 10 can compare the characteristic frequencies of the signal S with the characteristic frequencies of the signal R.

Finally, in a step 3008, the control circuit 22 or the external processing unit 10 can adapt the characteristic frequencies $f_1 ... f_n$ of the signal S. For instance, in one embodiment, the control circuit 22 or the external processing unit 10 can shift at least one frequency of the signal S in such a way that this frequency will correspond to the nearest peak (with comparable amplitude) in the signal R in such a way that the frequencies of the driving signal S will correspond to the characteristic frequencies of the tissue being treated.

For instance, Figures 10a and 10b show an example of a modified spectrum and the corresponding waveform of the driving signal S in the time domain. In particular, in the example considered, the initial harmonics (represented with reference to Figures 6a to 6e)

- $f_1 = 10$ Hz, $a_1 = 1$,
- $f_2 = 20$ Hz, $a_2 = 0.5$, and
- $f_3 = 30$ Hz, $a_3 = 0.25$

have been modified, reducing the amplitude $a_1$ of the first harmonic and increasing the frequency $f_2$ of the second harmonic, as follows:

- $f_1 = 10$ Hz, $a_1 = 0.5$;
- $f_2 = 25$ Hz, $a_2 = 0.5$; and
- $f_3 = 30$ Hz, $a_3 = 0.25$.

In general, the apparatus according to present disclosure may be used for all the treatments already mentioned in the document No. WO 2012/172504, in particular, for treatment of diabetic-foot ulcers, ulcers due to pressure and sores that are difficult to treat, for stimulation of the process of formation of bone callus, in pseudoarthroses and delayed consolidation, for stimulation of the process of osseointegration, in prevention and treatment of the peri-implantites, and for treatment of panniculopathy and remodelling of hypertrophic cicatricial outcomes of various nature.

For instance, the system according to the present disclosure may be used for at least one of the following applications that result from the hypotheses underlying its functioning:

- healing of wounds that are difficult to treat, ulcers due to pressure and diabetic-foot sores, with further reduction of the healing times as compared to the system described in the document No. WO 2012/172504;
- reduction of the neuropathic pain;
- prevention of complications of diabetes and general reduction of the hyperglycaemic states (thanks to the increased affinity of the membrane receptors coupled to G proteins);
- reduction of muscular spasticity of various origin (thanks to the increased current of Ca++ ions at the muscle-cell level);
- applications of cognitive stimulations in disability, in elderly persons, or in persons who practise sports;
- application in osseointegration of dental implants; prevention and treatment of the peri-implantitis;
- treatment of panniculopathy; and
- remodelling of hypertrophic cicatricial outcomes of various nature.

Of course, without prejudice to the principle of the invention, the details of construction and the embodiments may vary widely with respect to what has been described and illustrated herein purely by way of example, without thereby departing from the scope of the present invention, as defined by the ensuing claims.

## Claims

1. A system for therapeutic treatments with electromagnetic waves, comprising a diffuser (30) and a wave generator (10, 20) configured for generating a driving signal (S) for said diffuser (30) in order to generate said electromagnetic wave with specific frequency contributions, wherein said system is configured to generate said driving signal (S) on the basis of spectral content, which can be specified arbitrarily directly in the frequency domain, wherein said system comprises:

   - a digital-to-analog converter (224) configured for receiving digital data ($S_{Dk}$) at input and for supplying said driving signal (S) at output;

- a memory (102; 222) that contains discretized data ($f_1 ... f_n$, $a_1 ... a_n$) that identify the spectrum of said driving signal (S) in the frequency domain, wherein said discretized data ($f_1 ... f_n$, $a_1 ... a_n$) that identify the spectrum of said driving signal (S) in the frequency domain comprise data that identify a list of a plurality of harmonics ($f_1 ... f_n$) that said driving signal (S) should comprise; and

- at least one digital processing unit (10, 220) configured for:

a) receiving (1002; 2002) said discretized data that identify the spectrum of said driving signal (S) in the frequency domain from said memory (102; 222);

b) transforming (1004; 2004) said discretized data ($f_1 ... f_n$, $a_1 ... a_n$) that identify the spectrum of said driving signal (S) in the frequency domain into discretized data ($S_{Dk}$) that identify the waveform of said driving signal (S) in the time domain by determining a sequence of digital samples ($S_{Dk}$) via calculation (2004) of a sum of sinusoidal waves, wherein said sinusoidal waves have the frequencies of said plurality of harmonics ($f_1 ... f_n$), respectively; and

c) sending said digital samples ($S_{Dk}$) to said digital-to-analog converter (224);

**characterized in that** said processing unit (220) comprises:

- a single look-up table (2206) in which the amplitudes ($A_k$) corresponding to a given number of samples of a standard sinusoidal wave with a given frequency are stored,

- a plurality of counters ($2202_1 ... 2202_n$), each counter ($2202_1 ... 2202_n$) receiving at input a respective frequency ($f_1 ... f_n$) and providing at output a respective count value, wherein each counter ($2202_1 ... 2202_n$) represents a phase accumulator, which is incremented by a certain value at each iteration, where the value of the increment depends upon the ratio between the respective frequency of the required sinusoidal signal and the given frequency of the standard sinusoidal signal, wherein said look-up table (2206) is configured to receive sequentially one of the count values of the counters ($2202_1 ... 2202_n$) and provide the respective amplitude ($A_k(f_i)$); and

- a circuit (2230) configured to sum said amplitudes ($A_k(f_i)$), thereby calculating (2004) said sum of sinusoidal waves, wherein said sinusoidal waves have the frequencies of said plurality

of harmonics ($f_1 ... f_n$), respectively.

2. The system according to Claim 1, wherein said discretized data ($f_1 ... f_n$, $a_1 ... a_n$) that identify the spectrum of said driving signal (S) in the frequency domain comprises data that identify the respective amplitudes ($a_1 ... a_n$) of said plurality of harmonics ($f_1 ... f_n$) that said driving signal (S) should comprise.

3. The system according to any one of the preceding claims, wherein said at least one digital processing unit (10, 220) is configured for:

- transforming (1004; 2004) in real time said discretized data ($f_1 ... f_n$, $a_1 ... a_n$) that identify the spectrum of said driving signal (S) in the frequency domain into discretized data ($S_{Dk}$) that identify the waveform of said at least one driving signal (S) in the time domain.

4. The system according to any one of the preceding claims, wherein said at least one digital processing unit (10, 220) is configured for:

- varying (1016; 2012), during execution of a treatment program, said discretized data ($f_1 ... f_n$, $a_1 ... a_n$) that identify the spectrum of said driving signal (S) in the frequency domain.

5. The system according to Claim 3, wherein said system comprises a sensor (40) configured to supply a biofeedback signal (R), and wherein said at least one digital processing unit (10, 220) is configured for:

- varying (1016; 2012) said discretized data ($f_1 ... f_n$, $a_1 ... a_n$) that identify the spectrum of said driving signal (S) in the frequency domain as a function of said biofeedback signal (R).

6. The system according to any one of the preceding claims, wherein said system comprises:

- at least one lowpass filter (28) configured for filtering said driving signal (S) supplied by said digital-to-analog converter (224).

7. The system according to any one of the preceding claims, wherein said system is configured for generating a plurality of driving signals ($S_1$, $S_2$, $S_3$) for a plurality of diffusers (30a, 30b, 30c).

**Patentansprüche**

1. System für therapeutische Behandlungen mit elektromagnetischen Wellen, das einen Diffusor (30) und einen Wellengenerator (10, 20) umfasst, der so konfiguriert ist, dass er ein Treibersignal (S) für den Diffusor (30) erzeugt, um die elektromagnetische Welle mit spezifischen Frequenzbeiträgen zu erzeugen, wobei das System so konfiguriert ist, dass es das Treibersignal (S) auf der Grundlage eines spektralen Inhalts erzeugt, der willkürlich direkt im Frequenzbereich spezifiziert werden kann, wobei das System umfasst:

- einen Digital/Analog-Wandler (224), der so konfiguriert ist, dass er digitale Daten ($S_{Dk}$) am Eingang empfängt und das Treibersignal (S) am Ausgang liefert;
- ein Speicher (102; 222), der diskretisierte Daten ($f_1 ... f_n$, $a_1 ... a_n$) enthält, die das Spektrum des Treibersignals (S) in der Frequenzdomäne identifizieren, wobei die diskretisierten Daten ($f_1 ... f_n$, $a1 ... a_n$), die das Spektrum des Treibersignals (S) in der Frequenzdomäne identifizieren, Daten umfassen, die eine Liste einer Vielzahl von Oberschwingungen ($f_1 ... f_n$) identifizieren, die das Treibersignal (S) umfassen sollte; und
- mindestens eine digitale Verarbeitungseinheit (10, 220), die konfiguriert ist zum:

a) Empfangen (1002; 2002) der genannten diskretisierten Daten, die das Spektrum des genannten Treibersignals (S) im Frequenzbereich aus dem Speicher (102; 222) identifizieren;
b) Transformieren (1004; 2004) der diskretisierten Daten ($f_1 ... f_n$, $a_1 ... a_n$), die das Spektrum des Treibersignals (S) in der Frequenzdomäne identifizieren, in diskretisierte Daten ($S_{Dk}$), die die Wellenform des Treibersignals (S) in der Zeitdomäne identifizieren, durch Bestimmen einer Sequenz von digitalen Abtastwerten ($S_{Dk}$) durch Berechnung (2004) einer Summe von Sinuswellen, wobei die Sinuswellen jeweils die Frequenzen der Vielzahl von Oberschwingungen ($f_1 ... f_n$) haben; und
c) Senden der digitalen Abtastwerte ($S_{Dk}$) an den Digital/Analog-Wandler (224);

**dadurch gekennzeichnet, dass** die Verarbeitungseinheit (220) umfasst:

- eine einzelne Nachschlagetabelle (2206), in der die Amplituden ($A_k$), die einer gegebenen Anzahl von Abtastwerten einer Standardsinuswelle mit einer gegebenen Frequenz entsprechen, gespeichert sind,
- eine Mehrzahl von Zählern ($2202_1 ... 2202_n$), wobei jeder Zähler ($2202_1 ... 2202_n$) am Eingang eine jeweilige Frequenz ($f_1 ... f_n$) empfängt und am Ausgang einen jeweiligen Zählwert bereitstellt, wobei jeder Zähler ($2202_1 ... 2202_n$) einen

Phasenakkumulator darstellt, der bei jeder Iteration um einen bestimmten Wert inkrementiert wird, wobei der Wert des Inkrements von dem Verhältnis zwischen der jeweiligen Frequenz des erforderlichen sinusförmigen Signals und der gegebenen Frequenz des sinusförmigen Standardsignals abhängt, wobei die Nachschlagetabelle (2206) so konfiguriert ist, dass sie sequentiell einen der Zählwerte der Zähler ($2202_1 ... 2202_n$) empfängt und die jeweilige Amplitude ($A_k(f_i)$) bereitstellt; und
- eine Schaltung (2230), die so konfiguriert ist, dass sie die Amplituden ($A_k(f_i)$) summiert und dadurch die Summe der Sinuswellen berechnet (2004), wobei die Sinuswellen jeweils die Frequenzen der Vielzahl von Oberschwingungen ($f_1 ... f_n$) haben.

2. Das System nach Anspruch 1, wobei die diskretisierten Daten ($f_1 ... f_n$, $a_1 ... a_n$), die das Spektrum des Treibersignals (S) im Frequenzbereich identifizieren, Daten umfassen, die die jeweiligen Amplituden ($a_1 ... a_n$) der Vielzahl von Oberschwingungen ($f_1 ... f_n$) identifizieren, die das Treibersignal (S) umfassen sollte.

3. Das System gemäß einem der vorhergehenden Ansprüche, wobei die mindestens eine digitale Verarbeitungseinheit (10, 220) konfiguriert ist zum:

- Transformieren (1004; 2004) in Echtzeit der besagten diskretisierten Daten ($f_1 ... f_n$, $a_1 ... a_n$), die das Spektrum des besagten Treibersignals (S) im Frequenzbereich identifizieren, in diskretisierte Daten ($S_{Dk}$), die die Wellenform des besagten mindestens einen Treibersignals (S) im Zeitbereich identifizieren.

4. Das System gemäß einem der vorhergehenden Ansprüche, wobei die mindestens eine digitale Verarbeitungseinheit (10, 220) konfiguriert ist zum:

- Variieren (1016; 2012), während der Ausführung eines Behandlungsprogramms, der besagten diskretisierten Daten ($f_1 ... f_n$, $a_1 ... a_n$), die das Spektrum des besagten Treibersignals (S) im Frequenzbereich identifizieren.

5. Das System nach Anspruch 3, wobei das System einen Sensor (40) umfasst, der konfiguriert ist, ein Biofeedback-Signal (R) zu liefern, und wobei die mindestens eine digitale Verarbeitungseinheit (10, 220) konfiguriert ist zum:

- Variieren (1016; 2012) der diskretisierten Daten ($f_1 ... f_n$, $a_1 ... a_n$), die das Spektrum des Treibersignals (S) im Frequenzbereich als Funktion des Biofeedbacksignals (R) identifizieren.

**6.** Das System nach einem der vorhergehenden Ansprüche, wobei dieses System umfasst:

- mindestens ein Tiefpassfilter (28), der zum Filtern des von dem Digital/Analog-Wandler (224) gelieferten Treibersignals (S) konfiguriert ist.

**7.** System nach einem der vorhergehenden Ansprüche, wobei das System so konfiguriert ist, dass es eine Vielzahl von Treibersignalen ($S_1$, $S_2$, $S_3$) erzeugt für eine Vielzahl von Diffusoren (30a, 30b, 30c).

**Revendications**

**1.** Système pour traitements thérapeutiques au moyen d'ondes électromagnétiques, comprenant un diffuseur (30), et un générateur d'ondes (10, 20) configuré de manière à générer un signal d'excitation (S) pour ledit diffuseur (30) afin de générer ladite onde électromagnétique avec des contributions de fréquence spécifiques, dans lequel ledit système est configuré de manière à générer ledit signal d'excitation (S) sur la base d'un contenu spectral, qui peut être spécifié arbitrairement directement dans le domaine fréquentiel, dans lequel ledit système comprend :

- un convertisseur numérique-analogique (224) configuré de manière à recevoir des données numériques ($S_{Dk}$) en entrée, et à fournir ledit signal d'excitation (S) en sortie ;
- une mémoire (102 ; 222) qui contient des données discrétisées ($f_1 ... f_n$, $a_1 ... a_n$) qui identifient le spectre dudit signal d'excitation (S) dans le domaine fréquentiel, dans lequel lesdites données discrétisées ($f_1 ... f_n$, $a_1 ... a_n$) qui identifient le spectre dudit signal d'excitation (S) dans le domaine fréquentiel comprennent des données qui identifient une liste d'une pluralité d'harmoniques ($f_1 ... f_n$) que ledit signal d'excitation (S) devrait comprendre ; et
- au moins une unité de traitement numérique (10, 220) configurée de manière à :

a) recevoir (1002 ; 2002) lesdites données discrétisées qui identifient le spectre dudit signal d'excitation (S) dans le domaine fréquentiel en provenance de ladite mémoire (102 ; 222) ;
b) transformer (1004 ; 2004) lesdites données discrétisées ($f_1 ... f_n$, $a_1 ... a_n$) qui identifient le spectre dudit signal d'excitation (S) dans le domaine fréquentiel en des données discrétisées ($S_{Dk}$) qui identifient la forme d'onde dudit signal d'excitation (S) dans le domaine temporel, en déterminant une séquence d'échantillons numériques ($S_{Dk}$) par le biais du calcul (2004) d'une somme d'ondes sinusoïdales, dans lequel lesdites ondes sinusoïdales présentent les fréquences de ladite pluralité d'harmoniques ($f_1 ... f_n$), respectivement ; et
c) envoyer lesdits échantillons numériques ($S_{Dk}$) audit convertisseur numérique-analogique (224) ;

**caractérisé en ce que** ladite unité de traitement (220) comprend :

- une table de consultation unique (2206) dans laquelle sont stockées les amplitudes ($A_k$) correspondant à un nombre donné d'échantillons d'une onde sinusoïdale standard d'une fréquence donnée ;
- une pluralité de compteurs ($2202_1 ... 2202_n$), chaque compteur ($2202_1 ... 2202_n$) recevant en entrée une fréquence respective ($f_1 ... f_n$) et fournissant en sortie une valeur de comptage respective, dans lequel chaque compteur ($2202_1, ..., 2202_n$) représente un accumulateur de phase, qui est incrémenté d'une valeur donnée à chaque itération, où la valeur de l'incrément dépend du rapport entre la fréquence respective du signal sinusoïdal requis et la fréquence donnée du signal sinusoïdal standard, dans lequel ladite table de consultation (2206) est configurée de manière à recevoir séquentiellement l'une des valeurs de comptage des compteurs ($2202_1, ..., 2202_n$) et à fournir l'amplitude respective ($A_k(f_i)$) ; et
- un circuit (2230) configuré de manière à sommer lesdites amplitudes ($A_k(f_i)$), ce qui permet de calculer par conséquent (2004) ladite somme d'ondes sinusoïdales, dans lequel lesdites ondes sinusoïdales présentent les fréquences de ladite pluralité d'harmoniques ($f_1 ... f_n$), respectivement.

**2.** Système selon la revendication 1, dans lequel lesdites données discrétisées ($f_1 ... f_n$, $a_1 ... a_n$) qui identifient le spectre dudit signal d'excitation (S) dans le domaine fréquentiel comprennent des données qui identifient les amplitudes respectives ($a_1 ... a_n$) de ladite pluralité d'harmoniques ($f_1 ... f_n$) que ledit signal d'excitation (S) devrait comprendre.

**3.** Système selon l'une quelconque des revendications précédentes, dans lequel ladite au moins une unité de traitement numérique (10, 220) est configurée de manière à :

- transformer (1004 ; 2004) en temps réel lesdites données discrétisées ($f_1 ... f_n$, $a_1 ... a_n$) qui identifient le spectre dudit signal d'excitation (S) dans le domaine fréquentiel, en des données discrétisées ($S_{Dk}$) qui identifient la forme d'onde dudit au moins un signal d'excitation (S) dans le domaine temporel.

4. Système selon l'une quelconque des revendications précédentes, dans lequel ladite au moins une unité de traitement numérique (10, 220) est configurée de manière à :

   - faire varier (1016 ; 2012), pendant l'exécution d'un programme de traitement thérapeutique, lesdites données discrétisées ($f_1 ... f_n$, $a_1 ... a_n$) qui identifient le spectre dudit signal d'excitation (S) dans le domaine fréquentiel.

5. Système selon la revendication 3, dans lequel ledit système comprend un capteur (40) configuré de manière à fournir un signal de rétroaction biologique (R), et dans lequel ladite au moins une unité de traitement numérique (10, 220) est configurée de manière à :

   - faire varier (1016 ; 2012) lesdites données discrétisées ($f_1 ... f_n$, $a_1 ... a_n$) qui identifient le spectre dudit signal d'excitation (S) dans le domaine fréquentiel, en fonction dudit signal de rétroaction biologique (R).

6. Système selon l'une quelconque des revendications précédentes, dans lequel ledit système comprend :

   - au moins un filtre passe-bas (28) configuré de manière à filtrer ledit signal d'excitation (S) fourni par ledit convertisseur numérique-analogique (224).

7. Système selon l'une quelconque des revendications précédentes, dans lequel ledit système est configuré de manière à générer une pluralité de signaux d'excitation ($S_1$, $S_2$, $S_3$) pour une pluralité de diffuseurs (30a, 30b, 30c).

Fig. 1

Fig. 2

Fig. 3

Fig. 7

Fig. 4

Fig. 5

Fig. 6a

Fig. 6b

Fig. 6c

Fig. 6d

Fig. 6e

Fig. 10a

Fig. 10b

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012172504 A
- US 20050116742 A1
- US 20050033381 A1
- US 7897399 B

**Non-patent literature cited in the description**

- **PALL M.L.** Electromagnetic fields act via activation of voltage-gated calcium channels to produce beneficial or adverse effects. *J. Cell. Mol. Med.,* August 2013, vol. 17 (8), 958-65
- **ARUNA S. RAJAN et al.** Ion Channels and Insulin Secretion. *Diabetes Care,* 1990, vol. 13, 340-63
- **HALLIDAY, D. ; RESNICK, R.** Fundamentals of Physics. Wiley, 1981